# EUROPEAN PATENT SPECIFICATION

(11) **EP 1 569 650 B1**
(45) Date of publication and mention of the grant of the patent: **28.05.2008**
(21) Application number: 03732466.2
(22) Date of filing: 26.05.2003
(51) Int. Cl.: A61K 31/495, A61P 37/00

(54) **USE OF LEVOCETIRIZINE FOR THE TREATMENT OF PERSISTENT ALLERGIC RHINITIS**
VERWENDUNG VON LEVOCETIRIZIN ZUR BEHANDLUNG VON PERSISTIERENDER ALLERGISCHER RHINITIS
UTILISATION DE LEVOCETIRIZINE POUR LE TRAITEMENT DE LA RHINITE ALLERGIQUE PERSISTANTE

(30) Priority: 03.12.2002 EP 02080064
(43) Date of publication of application: 07.09.2005
(73) Proprietor: UCB FARCHIM S.A., 1630 Bulle (CH)
(72) Inventor: REVIRRON, Christophe, CH-1651 Villarvolard (CH)
(74) Representative: Lechien, Monique
(86) International application number: PCT/EP2003/005491
(87) International publication number: WO 2004/050094

(56) References cited:
- WO-A-94/06429
- "Xyzal (Levocetirizine) launched in England for Allergic Rhinitis" INTERNET, [Online] 1 October 2001 (2001-10-01), XP002240083 Retrieved from the Internet: <URL:http://www.pslgroup.com/dg/207766.htm http://www.pslgroup.com/dg/207766.htm> [retrieved on 2003-05-05]
- "Germany Approves Antihistamines Xyzal" INTERNET, [Online] 16 January 2001 (2001-01-16), XP002240084 Retrieved from the Internet: <URL:http://www.pslgroup.com/dg/1efc66.htm http://www.pslgroup.com/dg/1efc66.htm> [retrieved on 2003-05-05]
- GENSTHALER BM: "Levocetirizine: R-enantiomer against allergies" PHARMAZEUTISCHE ZEITUNG, vol. 146, no. 7, 15 February 2001 (2001-02-15), pages 35-36, XP001147797
- GRANT J ANDREW ET AL: "A double-blind, randomized, single-dose, crossover comparison of levocetirizine with ebastine, fexofenadine, loratadine, mizolastine, and placebo: suppression of histamine-induced wheal-and-flare response during 24 hours in healthy male subjects." ANNALS OF ALLERGY, ASTHMA & IMMUNOLOGY: OFFICIAL PUBLICATION OF THE AMERICAN COLLEGE OF ALLERGY, ASTHMA, & IMMUNOLOGY. UNITED STATES FEB 2002, vol. 88, no. 2, February 2002 (2002-02), pages 190-197, XP009010299 ISSN: 1081-1206
- GANDON J M ET AL: "Lack of effect of single and repeated doses of levocetirizine, a new antihistamine drug, on cognitive and psychomotor functions in healthy volunteers." BRITISH JOURNAL OF CLINICAL PHARMACOLOGY, vol. 54, no. 1, July 2002 (2002-07), pages 51-58, XP002240086 e=bjcp July, 2002 ISSN: 0306-5251
- BOUSQUET AND THE ARIA WORKSHOP GROUP: J ALLERGY CLIN IMMUNOL, vol. 108, 2001, pages S147-S152,
- THE ARIA GROUP IN COLLABORATION WITH THE WHO: "Management of allergic rhinitis and its impact on asthma - Pocket guide for physicians and nurses" StartDateMarker 2001, EndDateMarker * the whole document *
- BOUSQUET ET AL: "Characteristics...." CLIN EXP ALLERGY, vol. 35, 2005, pages 728-732,
- MULLOL ET AL: "management of persistent....." THERAPEUTICS AND CLINICAL RISK MANAGEMENT, vol. 4, no. 1, 2005, pages 265-271,
- BACHERT ET AL: "Levocetirizine....." J ALLERGY CLINI IMMUNOL, vol. 114, 2004, pages 838-844,

## Description

The present invention relates to the use of levocetirizine for the preparation of drugs effective for the treatment of the persistent allergic rhinitis.

International patent application 94/06429 describes the use of levocetirizine for the treatment of seasonal and perennial allergic rhinitis.

*A pocket guide for physicians and nurses (2001) The allergicmrhinitis and its impact on asthma workshop report in collaboration with the WHO* proposes the treatment of persistent rhinitis with oral H1-antihistamines (pg. 18 and 22). Levocetirizine is mentioned on pg. 15 as one of the new compounds of this class of therapeutic drugs.

It has now surprisingly been found that levocetirizine possesses therapeutic properties which render it particularly useful in the treatment of persistent allergic rhinitis. These activities are not observed in the dextrocetirizine.

The purpose of the invention concerns the treatment of persistent allergic rhinitis.

The present invention is based on the unexpected recognition that administration of pharmaceutical compositions comprising levocetirizine, or a pharmaceutically acceptable salt thereof to a patient treats the persistent allergic rhinitis.

The present invention encompasses a method for treating persistent allergic rhinitis which comprises administering to a patient a therapeutically effective amount of levocetirizine or a pharmaceutically acceptable salt thereof.

The present invention also encompasses the use of levocetirizine or a pharmaceutically acceptable salt thereof for the preparation of a medicament intended for the treatment of persistent allergic rhinitis.

The present invention relates to the use of levocetirizine or a pharmaceutically acceptable salt thereof for the preparation of a medicament intended for decreasing the symptoms of persistent allergic rhinitis and improving the quality of life.

In another aspect, the present invention relates to a method of treating in a patient persistent allergic rhinitis by administering an effective dose of levocetirizine or a pharmaceutically acceptable salt thereof.

The term "cetirizine" refers to the racemate of [2-[4-[(4 chlorophenyl)phenylmethyl]-1-piperazinyl]ethoxy]-acetic acid and its dihydrochloride salt which is well known as cetirizine dihydrochloride; its levorotatory and dextrorotatory enantiomers are known as levocetirizine and dextrocetirizine. Processes for preparing cetirizine, an individual optical isomer thereof or a pharmaceutically acceptable salt thereof have been described in European Patent 0 058 146, Great Britain Patent 2.225.320, Great Britain Patent 2.225.321, United States Patent 5,478,941, European Patent application 0 601 028, European Patent Application 0 801 064 and International Patent Application WO 97/37982.

The term "levocetirizine" as used herein means the levorotatory enantiomer of cetirizine. More precisely, it means that the active substance comprises at least 90% by weight, preferably at least 95% by weight, of one individual optical isomer of cetirizine and at most 10% by weight, preferably at most 5% by weight, of the other individual optical isomer of cetirizine. Each individual optical isomer may be obtained by conventional means, i.e., resolution from the corresponding racemic mixture or by asymmetric synthesis. Each individual optical isomer may be obtained from its racemic mixture by using conventional means such as disclosed in British patent application No. 2,225,321. Additionally, each individual optical isomer can be prepared from the racemic mixture by enzymatic biocatalytic resolution, such as disclosed in U.S. Patents No. 4,800,162 and 5,057,427.

The term "pharmaceutically acceptable salts" as used herein refers not only to addition salts with pharmaceutically acceptable non-toxic organic and inorganic acids, such as acetic, citric, maleic, succinic, ascorbic, hydrochloric, hydrobromic, sulfuric, and phosphoric acids and the like, but also its metal salts (for example sodium or potassium salts) or ammonium salts, the amine salts and the aminoacid salts. The best results have been obtained with levocetirizine dihydrochloride.

By patient, we understand children, adolescents and adults.

By the term "allergic rhinitis", we understand a symptomatic disorder of the nose induced by an IgE-mediated inflammation after allergen exposure of the membrane of the nose. Symptoms of allergic rhinitis include rhinorrhea, nasal obstruction, nasal itching, sneezing, ocular pruritis. The term "persistent allergic rhinitis", as used herein, refers to a disease when symptoms last more than 4 days per week and for more than 4 weeks. It is subdivided into mild and moderate-severe rhinitis. It is said "mild" when there are normal sleep, or no impairment of normal daily activities, sport, leisure, normal work and school, or no troublesome symptoms. It is said "moderate-severe" when there are abnormal sleep, or impairment of daily activities, sport, leisure, or problems caused at work or school, or troublesome symptoms.

A therapeutically effective amount of levocetirizine or a pharmaceutically acceptable salt thereof is used to treat or alleviate the effects of persistent allergic rhinitis. The dosage depends essentially on the specific method of administration and on the purpose of the treatment. The size of the individual doses and the administration program can best be determined based on an individual assessment of the relevant case. The methods required to determine the relevant factors are familiar to the expert.

A preferred daily dosage provides from about 0,0005 mg to about 2 mg of levocetirizine or a pharmaceutically acceptable salt thereof, per kg of body weight per patient. A particularly preferred daily dosage is from about 0,001 to about 2 mg per kg of body weight per patient. The best results have been obtained with a daily dosage from about 0,005 to 1 mg per kg of body weight per patient. The dosage may be administered once per day of treatment, or divided into smaller dosages, for examples 1 to 4 times a day, and preferably 1 to 3 times a day, and administrated over about a 24 hours time period to reach a total given dosage. Bests results have been obtained with an administration of a compositions of the invention are twice a day for children; and 5 mg once a day for adults. The exact dosages in which the compositions are administrated can vary according to the type of use, the mode of use, the requirements of the patient, as determined by a skilled practitioner. The exact dosage for a patient may be specifically adapted by a skilled person in view of the severity of the condition, the specific formulation used, and other drugs which may be involved.

Pharmaceutical compositions used according to the present invention may be administered by any conventional means. The routes of administration include intradermal, transdermal, slow release administration, intramuscular, oral and intranasal routes. Any other convenient route of administration can be used, for example absorption through epithelial or mucocutaneous linings.

The pharmaceutical forms according to the present invention may be prepared according to conventional methods used by pharmacists. The forms can be administered together with other components or biologicaly active agents, pharmaceutically acceptable surfactants, excipients, carriers, diluents and vehicles.

The pharmaceutical compositions of the invention include any conventional therapeutical inert carrier. The pharmaceutical compositions can contain inert as well as pharmacodynamically active additives. Liquid compositions can for example take the form of a sterile solution which is miscible with water. Furthermore, substances conventionally used as preserving, stabilizing, moisture-retaining, and emulsifying agents as well as substances such as salts for varying the osmotic pressure, substances for varying pH such as buffers, and other additives can also be present. If desired an antioxidant can be included in the pharmaceutical compositions. Pharmaceutical acceptable excipients or carriers for compositions include saline, buffered saline, dextrose or water. Compositions may also comprise specific stabilizing agents such as sugars, including mannose and mannitol. Carrier substances and diluents can be organic or inorganic substances, for example water, gelatine, lactose, starch, magnesium stearate, talc, gum arabic, polyalkylene glycol and the like. A prerequisite is that all adjuvants and substances used in the manufacture of the pharmaceutical compositions are nontoxic.

Pharmaceutical compositions can be administered by spray inhalation. Any conventional pharmaceutical composition for spray inhalation administration may be used. Another preferred mode of administration is by aerosol.

The pharmaceutical composition of the invention can also be formulated for topical application. The composition for topical application can be in the form of an aqueous solution, lotion or jelly, an oily solution or suspension or a fatty or emulsion ointment.

The pharmaceutical composition of the invention can also be used for slow prolonged release with a transdermal therapeutic system in polymer matrix or with an appropriate formulation for oral slow release.

The pharmaceutical compositions according to the present invention may also be administered orally or rectally. They may also be administered by nasal instillation, aerosols or in the form of unguents or creams. The pharmaceutical compositions which can be used for oral administration may be solid or liquid, for example, in the form of uncoated or coated tablets, pills, dragees, gelatine capsules, solutions, syrups and the like. For administration by the rectal route, the compositions containing the compounds of the present invention are generally used in the form of suppositories.

The pharmaceutical forms, such as tablets, drops, suppositories and the like, are prepared by conventional pharmaceutical methods. The compounds of the present invention are mixed with a solid or liquid, non-toxic and pharmaceutically acceptable carrier and possibly also mixed with a dispersing agent, a disintegration agent, a stabilizing agent and the like. If appropriate, it is also possible to add preservations, sweeteners, coloring agents and the like.

Preferably, the pharmaceutical compositions of the invention is administered in traditional form for oral administration, as film coated tablets, lozenges, dragees, and oral liquid preparation such as syrup.

Best results have been obtained with an oral dosage form, in particular liquid formulations such as syrup for children, and film-coated tablet for adults. For example, patients can receive 2 doses of 0,25 mg/kg (total daily dose : 0,50 mg/kg/day) of an oral solution of levocetirizine dihydrochloride 10 mg/ml per day; one ml of the solution contains 20 drops and one drop of levocetirizine dihydrochloride solution contains 0,5 mg.

As an Example of a composition according to the present invention, the following formulation of a film coated tablet is preferred: levocetirizine dihydrochloride, magnesium stearate, cellulose, lactose and silicon dioxide.

As an Example of a composition according to the present invention, the following formulation of a syrup is preferred: levocetirizine dihydrochloride, methyl- and propylparaben, saccharinum, and purified water.

Pharmaceutical compositions of the invention are useful to treat the persistent allergic rhinitis. These compositions can alleviate the effects of the persistent allergic rhinitis.

Another advantage of the invention is the ability of the process to improve quality of life and all symptoms of persistent allergic rhinitis.

The method of the invention is believed particularly suited to use in patients susceptible to suffer from persistent allergic rhinitis.

Another advantage of the invention is that levocetirizine dihydrochloride has an effect on rhinitis up to 6 months.

It is shown that levocetirizine dihydrochloride has an effect on quality of life up to 6 months.

It is shown that levocetirizine dihydrochloride has an effect on nasal congestion after 3 months. It lasts through 3 months.

The invention is further defined by reference to the following example.

### Example

The aim of the study relative to the clinical effect of levocetirizine dihydrochloride was to establish on the intention to treat (ITT population) whether a 6 month levocetirizine dihydrochloride treatment can improve the quality of life and clinical symptoms from adult patients suffering from persistent allergic rhinitis, when compared to placebo. For clinical symptoms, it was considered that a 1 point score reduction is clinically relevant. For health-related quality of life, it was considered that a 0.36 point score reduction is relevant. Secondary parameters of efficacy included different durations of treatment, different symptoms, different quality of life questionnaires, the incidence of co-morbidities suspected to be linked to allergic rhinitis and pharmaco-economic variables. The safety of this long-term treatment with levocetirizine dihydrochloride has also been evaluated.

The target population of this example consisted of adults aged more than 18 years suffering from persistent allergic rhinitis [WHO Initiative on Allergic Rhinitis and its Impact on Asthma (ARIA), 2000, pages S147-S149]. To be enrolled, the subjects needed to have sufficient rhinitis symptoms during the selection period. Excluded were patients with ENT or eye infection during the 2-weeks preceding initial visit.

The study was a prospective, randomized, double blind, parallel group, and placebo-controlled study with levocetirizine dihydrochloride.

The severity of clinical symptoms was rated by the T5SS (sneezing, rhinorrhea, nasal pruritus, ocular pruritus and nasal congestion) evaluated each by a score from 0 to 3. The impact on health related quality of life was measured using the Rhinoconjunctivitis Quality of Life Questionnaire (RQLQ) (E. JUNIPER and G.H. GUYATT, Development and testing of a new measure of health status for clinical trials in rhinoconjunctivitis, Clinical and Experimental Allergy 1991; 21:77-83; E. JUNIPER, Measuring Health Related Quality of Life in rhinitis, J. Allergy Clin. Immunol. 1997; 99:S742-9).

Study treatment lasted for 6 months. After the treatment period, patients entered a 1-week follow-up period.

The primary end-point for efficacy was a decrease of the T5SS over the first 4 weeks of at least 1 unit of score. The primary end-point for quality of life was a decrease of RQLQ after 4 weeks of at least 0.36 unit of the total score.

Secondary parameters of efficacy included the mean T5SS, the RQLQ and the SF-36 questionnaire at the different time points of the study, and the incidence and the duration of rescue medication over 6 months.

Exploratory parameters of efficacy included the mean of each individual rhinitis score, each RQLQ domain and each scale of the SF-36 questionnaire at the different time points of the study, the Global Evaluation Scale after 4 weeks and 6 months, the incidence of co-morbidities suspected to be linked to allergic rhinitis and the pharmaco-economic direct and indirect costs over 6 months.

At each of the eight visits, diary book entries (T5SS, RQLQ, SF-36, indirect cost pharmaco-economic parameters, concomitant medication, outpatient consultations and adverse events) were verified and transferred into the Clinical Record Form and direct cost pharmaco-economic parameters were recorded. Patients underwent a physical examination, including the measurement of vital signs. At the beginning and at the end of the study they also underwent a safety lab test, including pregnancy test for females, and at Visits 4 and 7, they filled-in a Global evaluation scale.

Adverse events were recorded by the patients on diary cards and discussed with the investigator at each visit. Serious adverse events had to be reported immediately.

Oral tablets of levocetirizine dihydrochloride (5 mg) and matching placebo, similar in appearance, shape and taste were used. The recommended study dosage was 1 tablet per day.

Sample size was based on 40% relative improvement over placebo in the RQLQ questionnaire, assuming an improvement from baseline for placebo of 0.9. For this questionnaire this corresponds to a difference of 0.36 vs. placebo.

The baseline characteristics of the two treatment groups, including demographic data, were comparable.

The study shows that treatment with levocetirizine dihydrochloride improves the symptoms of persistent allergic rhinitis (Difference of T5SS over the first 4 weeks: 1.14, p < 0.001; this difference being maintained over the whole study period) and the QOL (Change from baseline of the RQLQ Overall Score at first 4 weeks: 0.48, p < 0.001; this difference being maintained over the whole study period). A statistically significant improvement is also observed at all time points for sneezing, rhinorrhea, nasal pruritus and ocular pruritus. In addition, an improvement of nasal obstruction is observed, which becomes statistically different from 3 months onwards (Difference vs. placebo: 0.15, p = 0.009). Moreover, the long-term administration of levocetirizine did not involve particular safety concerns.

This study provides evidence of activity of levocetirizine in persistent allergic rhinitis. Levocetirizine is shown to be active on nasal obstruction after a long term treatment (equal or more than 3 months).

**Table I**

| Mean T5SS over the first four weeks of treatment | | | | | | | | | |
|---|---|---|---|---|---|---|---|---|---|
| (ITT population) | | | | | | | | | |
| Treatment | N | Baseline Mean | (SD) | Mean | (SD) | Adjusted mean^{(a)} | (SE) | Diff. vs. placebo^{(b)} | p-value^{(c)} |
| | | | | | | | | (95 % CI) | |
| Placebo | 271 | 8.90 | (2.26) | 6.61 | (2.47) | 6.56 | (0.15) | | |
| Lctz 5 mg | 276 | 9.02 | (2.28) | 5.53 | (2.52) | 5.43 | (0.15) | 1.14 [0.75, 1.52] | < 0.001 |

| | | | | | | | | | |
|---|---|---|---|---|---|---|---|---|---|
| ^{(a}) Mean adjusted for baseline score and country. ^{(b)} Placebo minus levocetirizine dihydrochloride (Lctz) 5mg. ^{(c}) p-value was obtained from an ANCOVA with baseline score as covariate and country and treatment as factors. | | | | | | | | | |

In table I it is shown that treatment with levocetirizine dihydrochloride improves the symptoms of persistent allergic rhinitis.

**Table II**

| Change from baseline of the RQLQ Overall Score after first 4 weeks of treatment | | | | | | | | | |
|---|---|---|---|---|---|---|---|---|---|
| (ITT population) | | | | | | | | | |
| | | Baseline | | Change | | | | | |
| Treatment | N | Mean | (SD) | Mean | (SD) | Adjusted Mean^{(a)} | (SE) | Diff. vs. placebo^{(b)} | p-value^{(c)} |
| | | | | | | | | (95 % CI) | |
| Placebo | 252 | 3.06 | (0.94) | -0.99 | (1.25) | -1.01 | (0.07) | | |
| Lctz 5 mg | 257 | 3.04 | (0.92) | -1.50 | (1.18) | -1.49 | (0.07) | 0.48 [0.29, 0.67] | < 0.001 |

| | | | | | | | | | |
|---|---|---|---|---|---|---|---|---|---|
| ^{(a)} Mean adjusted for baseline score and country. ^{(b)} Placebo minus levocetirizine dihydrochloride 5mg. ^{(c)} p-value was obtained from an ANCOVA with baseline score as covariate and country and treatment as factors. | | | | | | | | | |

In table II it is shown that treatment with levocetirizine dihydrochloride improves the quality of life.

**Table III**

| Nasal congestion symptoms evaluated over the 24 hours, over the first week and first 4 weeks, 3, 4.5 and 6 months of treatment | | | | | | | | | | |
|---|---|---|---|---|---|---|---|---|---|---|
| (ITT population) | | | | | | | | | | |
| Period | Treatment | N | Baseline | | Mean | (SD) | Adjusted Mean^{(a)} | (SE) | Diff. vs. | p-value^{(c)} |
| | | | Mean | (SD) | | | | | (95% CI)^{(b)} | |
| Week 1 | Placebo | 270 | 1.85 | (0.71) | 1.64 | (0.77) | 1.65 | (0.04) | | |
| | Lctz 5 mg | 271 | 1.90 | (0.69) | 1.61 | (0.83) | 1.58 | (0.04) | 0.07 [-0.04; 0.18] | 0.203 |
| First | Placebo | 271 | 1.85 | (0.71) | 1.49 | (0.74) | 1.48 | (0.04) | | |
| 4 weeks | Lctz 5 mg | 276 | 1.91 | (0.69) | 1.44 | (0.78) | 1.40 | (0.04) | 0.08 [-0.02; 0.19] | 0.123 |
| 3 months | Placebo | 270 | 1.85 | (0.71) | 1.33 | (0.74) | 1.31 | (0.04) | | |
| | Lctz 5 mg | 276 | 1.91 | (0.69) | 1.22 | (0.78) | 1.16 | (0.04) | 0.15 [0.04; 0.26] | 0.009 |
| 4.5 months | Placebo | 270 | 1.85 | (0.71) | 1.29 | (0.74) | 1.27 | (0.04) | | |
| | Lctz 5 mg | 276 | 1.91 | (0.69) | 1.17 | (0.77) | 1.11 | (0.04) | 0.15 [0.04; 0.26] | 0.007 |
| 6 months | Placebo | 270 | 1.85 | (0.71) | 1.26 | (0.74) | 1.24 | (0.04) | | |
| | Lctz 5 mg | 276 | 1.91 | (0.69) | 1.13 | (0.76) | 1.08 | (0.04) | 0.16 [0.05;0.27] | 0.005 |

| | | | | | | | | | | |
|---|---|---|---|---|---|---|---|---|---|---|
| ^{(a)} On study Mean adjusted for baseline score and country. ^{(b)} Placebo minus levocetirizine dihydrochloride. ^{(c)} p-value was obtained from an ANCOVA with baseline score as covariate and country and treatment as factors. | | | | | | | | | | |

In table III it is shown that levocetirizine dihydrochloride is shown to be active on nasal obstruction after a long term treatment.

The following abbreviations are used in the example:
- T5SS: Total 5 Symptoms Score
- ITT: Intention-to-Treat
- N: Number
- SD: Standard Deviation
- SE: Standard Error of the Mean
- Diff.: Difference
- vs.: versus
- CI: Confidence Interval
- P: probability that the observed difference is only by chance
- RQLQ: Rhinoconjunctivitis Quality of Life Questionnaire
- ANCOVA: Analysis of Covariance
- ENT: Ear-Nose-Throat
- SF-36: Medical Outcomes Survey Short Form 36
- Lctz: levocetirizine dihydrochloride.

A long duration of effect is noted. The positivity of the trial is due to the lack of tachyphylaxis, i.e there's no "adjustement" of the dosing schedule needed during 6 months. The recommended dosage is effective constantly throughout the trial.

An improvement of quality of life (QoL) is clearly noted during the trial. It is central to ARIA. This is the first time that a drug is able to change the QoL of patients for such a long duration. This is as close as possible to a "disease modifying" effect.

Nasal congestion is treated during the trial. Interestingly, nasal congestion is a symptom relief that appear during the trial, i.e the effect is gradual, this is congruent with the observation that QoL is improving.

It is demonstrated that levocetirizine dihydrochloride is able to treat persistent rhinitis as long as it is administered, but also able to modify daily activities of patients, going beyond the simple symptom relief observed in short duration trials so far.

## Claims

1. Use of levocetirizine or a pharmaceutically acceptable salt thereof for the preparation of a medicament intended for treating persistent allergic rhinitis or for decreasing the symptoms of persistent allergic rhinitis and improving the quality of life.

2. Use according to claim 1 wherein the medicament is intended for treating rhinorrhea.

3. Use according to claim 1 wherein the medicament is intended for treating nasal obstruction.

4. Use according to claim 1 wherein the medicament is intended for treating nasal itching.

5. Use according to claim 1 wherein the medicament is intended for treating sneezing.

6. Use according to claim 1 wherein the medicament is intended for treating ocular pruritis.

7. Use according to any one of claims 1 to claim 6, wherein the salt is the levocetirizine dihydrochloride.

8. Use according to any one of claims 1 to 7, wherein the medicament is adapted for administration of a daily dosage from about 0.0005 mg to about 2 mg of said levocetirizine or said pharmaceutically acceptable salt thereof, per kg of body weight per patient.

## Patentansprüche

1. Verwendung von Levocetirizin oder einem pharmazeutisch verträglichen Salz davon für die Herstellung eines Medikaments, das für die Behandlung von persistierendem allergischem Schnupfen oder für die Verminderung der Symptome von persistierendem allergischem Schnupfen und zur Verbesserung der Lebensqualität vorgesehen ist.

2. Verwendung nach Anspruch 1, wobei das Medikament für die Behandlung von Rhinorrhea vorgesehen ist.

3. Verwendung nach Anspruch 1, wobei das Medikament für die Behandlung von nasaler Obstruktion vorgesehen ist.

4. Verwendung nach Anspruch 1, wobei das Medikament für die Behandlung von Nasenjucken vorgesehen ist.

5. Verwendung nach Anspruch 1, wobei das Medikament für die Behandlung von Niesen vorgesehen ist.

6. Verwendung nach Anspruch 1, wobei das Medikament für die Behandlung von okularer Pruritis vorgesehen ist.

7. Verwendung nach einem der Ansprüche 1 bis Anspruch 6, wobei das Salz das Levocetirizin-Dihydrochlorid ist.

8. Verwendung nach einem der Ansprüche 1 bis 7, wobei das Medikament zur Verabreichung einer täglichen Dosierung von etwa 0,0005 mg bis etwa 2 mg von dem Levocetirizin oder dem pharmazeutisch verträglichen Salz davon, pro kg Körpergewicht pro Patient, angepasst ist.

## Revendications

1. Utilisation de la lévocétirizine ou de l'un de ses sels acceptables pharmaceutiquement pour la préparation d'un médicament destiné à traiter la rhinite allergique persistante ou à diminuer les symptômes de la rhinite allergique persistante et à améliorer la qualité de vie.

2. Utilisation suivant la revendication 1, dans laquelle le médicament est destiné à traiter la rhinorrhée.

3. Utilisation suivant la revendication 1, dans laquelle le médicament est destiné à traiter l'obstruction nasale.

4. Utilisation suivant la revendication 1, dans laquelle le médicament est destiné à traiter la démangeaison nasale.

5. Utilisation suivant la revendication 1, dans laquelle le médicament est destiné à traiter l'éternuement.

6. Utilisation suivant la revendication 1, dans laquelle le médicament est destiné à traiter le prurit oculaire.

7. Utilisation suivant l'une quelconque des revendications 1 à 6, dans laquelle le sel est le dichlorhydrate de lévocétirizine.

8. Utilisation suivant l'une quelconque des revendications 1 à 7, dans laquelle le médicament est conçu pour l'administration d'une posologie quotidienne d'environ 0,0005 mg à environ 2 mg de la lévocétirizine ou de l'un de ses sels acceptables pharmaceutiquement, par kg de poids corporel du patient.
